# EUROPEAN PATENT SPECIFICATION

(11) **EP 1 105 508 B1**
(45) Date of publication and mention of the grant of the patent: **04.09.2002**
(21) Application number: 99944420.1
(22) Date of filing: 17.08.1999
(51) Int. Cl.: C12N 15/74, C12N 15/11, C12N 15/62, C12P 21/02

(54) **METHOD FOR PURIFYING BIOMOLECULE OR PROTEIN COMPLEXES**
VERFAHREN ZUR REINIGUNG VON BIOMOLEKÜL- ODER PROTEINKOMPLEXEN
PROCEDE DE PURIFICATION DE BIOMOLECULES OU DE COMPLEXES DE PROTEINES

(30) Priority: 17.08.1998 EP 98115448
(43) Date of publication of application: 13.06.2001
(60) Divisional application: 02004765.0
(73) Proprietor: EUROPÄISCHES LABORATORIUM FÜR MOLEKULARBIOLOGIE (EMBL), D-69117 Heidelberg (DE)
(72) Inventor: SERAPHIN, Bertrand, D-69168 Wiesloch (DE); RIGAUT, Guillaume, D-69126 Heidelberg (DE)
(74) Representative: Dey, Michael, Dr.
(86) International application number: EP9906022
(87) International publication number: WO00009716

(56) References cited:
- WO-A-96/40943
- SENGER B ET AL.: "Mtr10p functions as a nuclear import receptor for the mRNA-binding protein Npl3p" EMBO JOURNAL, vol. 17, no. 8, 1 August 1998 (1998-08-01), pages 2196-2207, XP002090021 cited in the application
- ZHENG C ET AL: "A new expression vector for high level protein production, one step purification and direct isotopic labeling of calmodulin-binding peptide fusion proteins" GENE, vol. 186, no. 1, 20 February 1997 (1997-02-20), page 55-60 XP004054879
- PANAGIOTIDIS C A ET AL: "pALEX, a dual-tag prokaryotic expression vector for the purification of full-length proteins" GENE, vol. 164, no. 1, 1995, page 45-47 XP004041915

## Description

The present invention relates to a method for purifying protein and/or biomolecule complexes.

Protein expression and purification methods are essential for studying the structure, activities, interactions with other proteins, nucleic acids etc. of proteins of interest. Methods that are currently available use systems such as bacteria or cells transfected with expression vectors or infected with bacculovirus.

In order to study individual proteins a first requirement is to obtain sufficient amounts of that particular protein to be able to carry out biological and biochemical analyses such as activity tests, interaction assays, structure determination and the like. For this purpose the genes coding for the proteins of interest are cloned into vectors that allow the expression of those proteins in suitable host cells. Usually the proteins are expressed at high levels. This over-expression leads to the generation of large amounts of protein but often has the disadvantage of yielding insoluble protein which is present in so-called inclusion bodies in the cells. The over-expressed protein then has to be resolubilized before analysis. Panagiotidis and Silverstein (Gene, 164 (1995), 45-47) describe the purification of over-expressed proteins via fusion to a dual tag in E.coli, resulting in insoluble proteins, which then have to be renaturized again.

Although such methods work well for conventional protein detection methods based on weight analysis (polyacrylamide gels, Western blots, etc.) of the expressed protein, they are not suitable for other studies and for assays on protein complexes.

Currently used protocols for over-expressing proteins are normally carried out in host cells in which the protein of interest is not normally expressed, for example, when eukaryotic proteins are expressed in bacteria. Apart from the insolubility, proteins expressed in this manner often show a lack of proper post-transcriptional or post-translational modification, such as correct processing or glycosylation.

Another disadvantage is that the expression of one subunit of a complex, even in a homologous system, might not lead to increased production of all the complex components and in some extreme cases can even result in mistargeting of that protein of interest to an aberrant complex (e.g. the proteasome, Swafield et al., 1996, Nature 379, 658).

Purification of proteins expressed at their basal level is therefore indispensable in many cases but appropriate purification protocols are not easily available because of the huge biochemical knowledge required to establish a suitable protocol. Developing a purification scheme requires assessing the chemical and physical properties of the target protein by a trial and error process, making it tedious and time-consuming because such analyses have to be repeated for each protein.

Affinity purification methods for the purification of proteins are known. Kits and apparatuses for affinity chromatography are commercially available. Usually, a fusion protein of a polypeptide of interest plus an affinity tag is expressed in a system such as one of the above-mentioned expression systems, the fusion protein is extracted and applied to support material such as a resin matrix packed in an affinity column which is coated with a material that specifically binds the affinity tag. After binding of the target protein to the matrix via the affinity tag unbound substances can be removed simply by washing the matrix. The fusion protein can then be eluted off the matrix using chemical agents or specific temperature or pH conditions. Since the affinity tags usually bind with high affinity strong conditions are needed for the subsequent elution wich can often destroy, damage or denature the protein of interest.

Affinity tags possess groups or moieties which are capable of binding to a specific binding partner with high affinity. Various affinity tags are known in the art and have been widely used for the purification of proteins. Examples are the IgG binding domains of protein A of Staphylococcus aureus, glutathione-S-transferase (GST), maltose binding protein, cellulose binding domain, polyarginine, polycysteine, polyhistidine, polyphenylalanine, calmodulin or calmodulin binding domains. These bind with high affinity to an appropriate matrix which is covered with the specific binding partner. In the case of protein A, IgG-coated sepharose has been used for affinity chromatography of fusion proteins possessing a protein A domain (Senger et al., 1998, EMBO J., Vol.17, 2196-2207). Other examples are discussed in Sassenfeld, TIBTECH, 1990, p.88. A plasmid vector containing a cassette encoding a calmodulin binding peptide is available from Stratagene.

Normally, fusion proteins are tagged with only one affinity tag and are purified in a single purification step. This often leads to problems due to remaining contaminants. Another limitation of most of the conventional methods is that they are adapted for expression of the proteins in bacteria only. W096/40943 discloses a method of expressing fusion proteins in gram-positive bacteria either anchored to the membrane or in secreted form. The anchored proteins are cleaved off using TEV protease and subsequently affinity purified via an affinity tag.

Often the affinity tag is removed from the fusion protein after the affinity purification step by the action of a specific protease such as the TEV protease. This means however, that the purified fractions contain substantial amounts of this protease (Senger et al. 1998) which severely limits the applications of such protein preparations.

It is therefore an object of the present invention to provide a purification/detection method for proteins and/or biomolecule complexes which eliminates the disadvantages of the currently known methods and allows efficient purification not only of affinity tagged target proteins as fusion proteins but also of other substances that are capable of associating with these proteins.

One method according to the invention for purifying biomolecule and/or protein complexes comprises the following steps:
(a) providing an expression environment containing one or more heterologous nucleic acids encoding one or more subunits of a biomolecule complex, the subunits being fused to at least two different affinity tags, one of which consists of one or more IgG binding domains of Staphylococcus protein A,
(b) maintaining the expression environment under conditions that facilitate expression of the one or more subunits in a native form as fusion proteins with the affinity tags, and under conditions that allow the formation of a complex between the one or more subunits and possibly other components capable of complexing with the one or more subunits,
(c) detecting and/or purifying the one or more subunits by a combination of at least two different affinity purification steps each comprising binding the one or more subunits via one affinity tag to a support material capable of selectively binding one of the affinity tags and separating the one or more subunits from the support material after substances not bound to the support material have been removed.

An alternative method of the invention which is particularly suitable for detecting and/or purifying protein or biomolecule complexes is a method comprising the steps:
(a) providing an expression environment containing one or more heterologous nucleic acids encoding at least two subunits of a biomolecule complex, each being fused to at least one of different affinity tags, one of which consists of one or more IgG binding domains of Staphylococcus protein A,
(b) maintaining the expression environment under conditions that facilitate expression of the two or more subunits in a native form as fusion proteins with the affinity tags, and under conditions that allow the formation of a complex between the two or more subunits and possibly other components capable of complexing with the two or more subunits,
(c) detecting and/or purifying the complex by a combination of at least two different affinity purification steps each comprising binding the two or more subunits via one affinity tag to a support material capable of selectively binding one of the affinity tags and separating the complex from the support material after substances not bound to the support material have been removed.

For the purpose of this invention, a biomolecule can be a protein, peptide or a nucleic acid or other biomolecule. A biomolecule complex denotes a complex of at least two biomolecules, preferably at least one protein associated either with other proteins which are then called subunits or with other substances which can for example be nucleic acids. The biomolecule complexes can be natural ones such as nuclear snRNPs or antigen-antibody complexes, or they can be artificial ones such as mutant DNA binding proteins associated with mutant target DNAs. Any complex molecule comprising as one or more subunits a polypeptide or subunit expressed according to the invention and/or further comprising other components which associate in a manner stable enough not to be dissociated by the affinity steps is a biomolecule complex that can be detected and/or purified by the method of the invention. A protein complex generally devotes a complex between protein subunits.

The nucleic acid sequence of at least one of the subunits of the protein complex to be purified must be known or at least available so that it can be cloned into a nucleic acid which is suitable to drive expression in the appropriate host cells or cell-free expression systems.

The heterologous nucleic acid driving the expression of the one or more subunits of the protein complex to be purified according to the invention thus contains appropriate sequences that allow it to be maintained in the chosen host cell or cell-free system, such as a promoter and, if necessary, other control sequences such as enhancers and poly A sites.

In principle any host cell that is compatible with the heterologous nucleic acid from which the one or more subunits are to be expressed is suitable as an expression environment. These cells can be prokaryotic cells such as bacteria etc. or eukaryotic cells such as yeast, fungi or mammalian cells. Preferably, the subunits or protein complex to be purified is expressed in its natural host. Since this method is very efficient, the protein subunits are preferably expressed at their basal levels. This has the advantage of avoiding the formation of inclusion bodies and also reduces the risk of toxic effects on the cell that large amounts of certain proteins may have. Furthermore, this avoids purifying excess protein subunits that are not assembled into a complex or that are assembled into aberrant complexes (see above).

After the heterologous nucleic acid encoding the fusion protein has been introduced into a chosen host cell the cell is cultured under conditions which allow the expression of the fusion protein(s).

As already mentioned, the transcriptional control sequences are preferably selected so that the fusion protein is not over-expressed but is expressed at basal levels in the cell. This serves to ensure that the protein is expressed in a native form. Native form means in this context that a correct or relatively close to natural three-dimensional structure of the protein is achieved, i.e. the protein is folded correctly. More preferably, the protein will also be processed correctly and show normal post-transcriptional and post-translational modification. The correct folding is of great importance especially when the expressed polypeptide is a subunit of a protein complex because it will bind to the other subunits of the complex only when it is present in its native form. However, it is also possible to express mutant proteins. These can also have a native conformation. Such mutant subunits can, for example, be used to purify mutant complexes, i.e. complexes that contain some other mutated subunits.

Depending on the subunit to be purified, the fusion protein is expressed intracellularly or secreted into the culture medium. Alternatively, it might be targeted to other cell compartments such as the membrane. Depending on the protein an appropriate method is used to extract the fusion protein from the cells and/or medium. When a fusion protein is expressed which is targetted to a certain subcellular location, e.g. the membrane of cell organelles or the cell membrane, these organelles or the cells themselves can be purified via the binding of these membrane proteins. It is also possible to purify cells or cell organelles via proteins naturally expressed on their surface which bind to the fusion protein of the invention.

Further, it is possible to purify biomolecule or protein complexes/subunits or other substances that are capable of binding to or complexing with the fusion protein generated according to the invention. These substances can bind to fusion protein either directly or via linker mediators. Linker mediators in this context may be anything which is capable of binding two or more biomolecules so that these biomolecules are then part of a complex although they may not be directly associated with each other.

According to the invention it is also possible to use cell-free systems for the expression of the subunits. These must provide all the components necessary to effect expression of proteins from the nucleic acid such as transcription factors, enzymes, ribosomes etc. In vitro transcription and translation systems are commercially available as kits so that it is not necessary to describe these systems in detail (e.g. rabbit reticulocyte lysate systems for translation). A cell-free or in vitro system should also allow the formation of complexes.

For the purification according to the invention it is preferable to employ affinity chromatography on affinity columns which contain a matrix coated with the appropriate binding partner for the affinity tag used in that particular purification step.

In accordance with the method of this invention two affinity steps are carried out. Basically each affinity step consists of a binding step in which the previously extracted one or more subunits are bound via one of their affinity tags to a support material which is covered with the appropriate binding partner for that affinity tag. Then unbound substances are removed and finally the subunit to be purified is recovered from the support material. This can be done in two ways. The first possibility is to simply use conventional elution techniques such as varying the pH or the salt or buffer concentrations and the like depending on the tag used. The second possibility is to release the subunit to be purified from the support material by proteolytically cleaving off the affinity tag bound to the support. This way, the subunit can be recovered in the form of a truncated fusion protein or, if all affinity tags have been cleaved off, as the target subunit itself.

According to one embodiment of the present invention a fusion protein of a single subunit plus two different affinity tags is expressed, wherein one of the tags comprises one or more IgG binding domains of protein A of Staphylococcus aureus.

More preferably, a specific proteolytic cleavage site is present in the fusion protein between the one or more subunits and the one or more affinity tags so that proteolytic cleavage allows the removal of at least one of the affinity tags, especially the IgG binding domains of protein A.

Proteolytic cleavage can be carried out by chemical means or enzymatically.

The proteoloytic cleavage site that is used to cleave off one of the affinity tags is preferably an enzymatic cleavage site. There are several proteases which are highly specific for short amino acid sequences which they will cleave. One of these is a specific cleavage site of Tobacco Etch Virus (TEV), which is cleaved by the TEV protease NIA. Recombinant TEV protease is available from Gibco BRL. The TEV cleavage site is preferably used as the cleavage site to remove the protein A domains from the fusion protein.

Even more preferably, the affinity step using protein A binding to IgG is carried out first by binding the one or more subunits via the one or more IgG binding domains of Staphylococcus to a support material capable of specifically binding the latter, removing substances not bound to the support material and separating the one or more subunits from the support material by cleaving off the IgG binding domains via the specific proteolytic cleavage site, and then another affinity tag is used to purify the protein further via a conventional elution step comprising binding the one or more subunits via another affinity tag to a second support material capable of specifically binding the latter, removing substances not bound to the support material and separating the one or more subunits from the support material.

When the proteins are present at low concentrations in the expression environment and on the support material, a large amount of protease is required to release the bound material from the support material. In other words, when the substrate concentration is low a high level of enzyme is required to drive an efficient proteolytic reaction. The second purification step is then important to remove remaining contaminants and the protease. Removal of the protease is preferable in order to eliminate any negative influences of the proteolytic activity on the preparation.

However, in some cases it may be desirable to remove all the affinity tags in which case it is also possible to utilise two or more different proteolytic cleavage sites for the separation of the one or more subunits of interest from the support material.

The method according to the invention not only facilitates efficient purification of proteins of interest but also allows fishing for and detecting components present in complexes with which the polypeptides or subunits are associated or complexed either directly or indirectly, e.g. molecules such as linker mediators. This would allow selective fishing for certain substances which may be potential drugs even from complex mixtures.

According to a second embodiment of the invention it is possible not only to detect or purify the subunit containing fusion proteins expressed but also other substances that are capable of associating with the proteins expressed in a direct way, i.e. by directly binding to the fusion protein, or indirectly via other molecules to form biomolecule complexes. If a fusion protein of a subunit of a biomolecule or protein complex is purified according to the invention the affinity steps are chosen so that other complex components which have bound to the fusion protein are still associated with the subunit after the purification steps so that they can be detected/purified as well.

The biomolecule complexes can be formed in the expression environment such as cellular complexes. Alternatively, other complex components may be added to the subunits already expressed to form complexes in vitro or may even be added when the subunit containing fusion protein is already bound to a support material in an affinity step.

It is also possible to express two (or more) subunits of the same complex each as a fusion protein with a different affinity tag. When the subunits associate they can be detected/purified possibly together with other complex components by a series of affinity steps in which each time the complex is bound via a differently tagged subunit. The two or more affinity tags can be fused with a single subunit of a complex or with two or more subunits which bind to each other or are simply present in the same complex.

The purification steps can be carried out as described above.

Some subunits are present in more than one complex so that the components of all complexes can be purified.

If one is interested in a single complex A one can also subtract other complexes B that also contain one of the subunits of A by fusing that subunit of A to one tag and fusing a subunit unique to B with a different tag. The tagged subunit of B will bind to a specific support material. If the fraction not bound to that support material is used in the second affinity purification step, complex B will no longer be present because it was removed (subtracted) in the first affinity step. Many similar scenarios can be envisaged and designed by a person skilled in the art.

Further affinity tags in addition to the IgG binding domains that can be used in accordance with the present invention can be any conventional affinity tag. Preferably, the second affinity tag consists of at least one calmodulin binding peptide (CBP). Calmodulin binding peptide as an affinity tag has been described and is commercially available (Stratagene). When a calmodulin binding peptide is used the corresponding purification step is carried out using a support material that is coated with calmodulin. The calmodulin binding peptide tag binds to calmodulin in the presence of low concentrations of calcium. It can subsequently be eluted using a chemical agent such as a chelating agent like EGTA. Preferably, around 2 mM EGTA is added for the elution step.

The following Examples and Figures serve to illustrate the invention and its practical application.

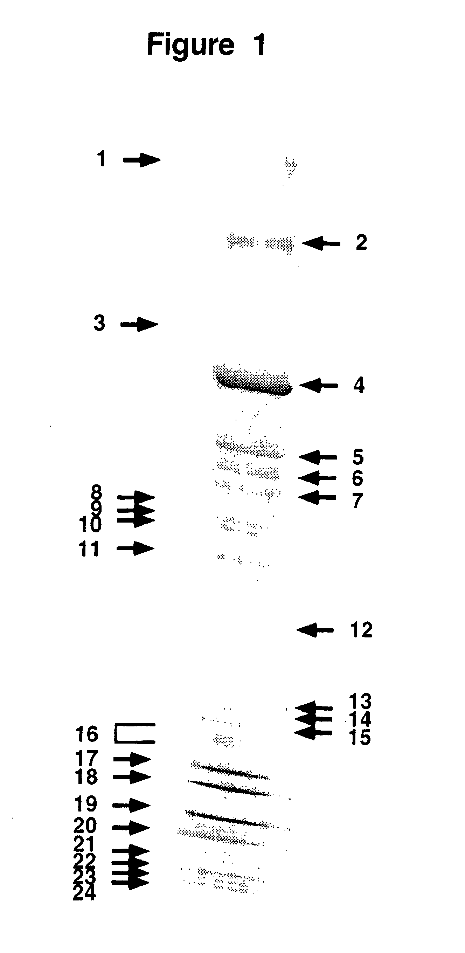

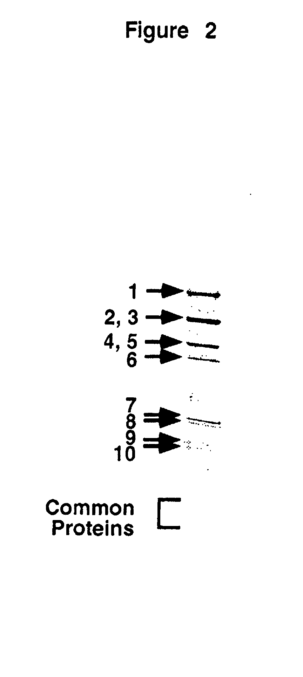

- Fig.1: shows a Coomassie stained gel depicting the fractionated proteins of a yeast RNA-protein complex. Proteins identified by mass spectrometry are labeled 1-24. Bands 1, 3, 8-11, 16-24 were expected in this complex. Bands 2, 4-7 represent proteins that are likely candidates for true complex component given their sequence. Bands 12-14 represent potential contaminants (ribosomal proteins). Band 15 is a trace amount of TEV protease that remained in this particular preparation. This is not generally the case (see Fig.2 for example). Bands 4-6 originate from the same gene and might represent alternative translation products or degradation products. Band 16 is a mixture and contains, in addition to a bona fide complex protein, a contaminating ribosomal protein.
- Fig.2: shows a Coomassie stained gel depicting the fractionated proteins moiety of the yeast U1 snRNP. The 10 specific proteins were identified by mass spectrometry. Compare with the silver stained gel obtained following a purification using a cap affinity step and a Ni-NTA column reported by Neubauer et al., (Proc.Natl.Acad.Sci USA 1997, 94, 385-390). Note in particular the low level of contaminants in this purification.

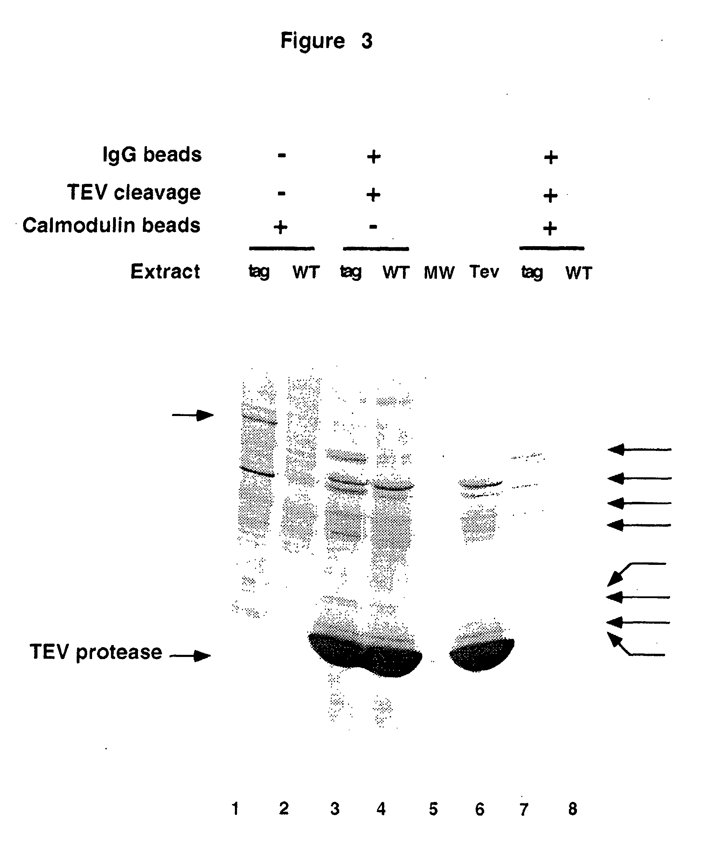

- Fig.3: shows a Coomassie stained gel in which purified U1 snRNP has been analysed using either the CBP binding/EGTA elution alone (lane 1), the Protein A binding/TEV elution alone (lane 3) or both steps (lane 7). Arrows on the right point to the U1 snRNP specific proteins. Lanes 2, 4 and 8 show the background signal obtained using each of the single steps or the two step procedure from an extract without tagged protein, demonstrating the requirement for two steps to get a pure material. Lane 6 is a molecular weight marker. Lane 7 is the TEV protease that can also be seen as an abundant contaminant (even though only the required amount of protease was used) in lanes 3 and 4. This demonstrates again the need for a second purification step.

### Examples

### Example 1

### Purification of protein complexes from yeast

A vector encoding a fusion of a yeast protein to the CBP-TEV-Protein A double tag was constructed using standard methods. The fusion protein is one subunit of a protein complex of yeast containing 24 subunits in total. The plasmid was transformed into yeast cells and a 2 L culture of cells expressing the protein was prepared. Proteins were extracted from the cultured cells using a French press. The complex was purified by binding to IgG-linked beads, eluting by TEV protease cleavage, binding of the eluted material on calmodulin containing beads followed by elution with EGTA. All steps were carried out at 0-4°C, excepted for TEV cleavage.

The first affinity step (IgG step) was performed as follows:
200 µl of IgG-Sepharose bead suspension (Pharmacia 17-0969-01) were washed in an Econocolumn with 5 ml of IPP 150-IgG buffer (10 mM Tris-CI pH 8.0, 150 mM NaCI, 0.1% NP40). 10 ml extract, corresponding approximately to 2 L of yeast culture, were adjusted to IPP 150-IgG buffer concentrations in Tris-CI pH 8.0, NaCI and NP40. This extract solution was mixed with the 200 µl of IgG-Sepharose beads and rotated in the Econocolumn for 2 hours. The unbound fraction was discarded and beads with bound material were washed first with 30 ml IPP 150-IgG buffer followed by 10 ml TEV cleavage buffer (10 mM Tris-CI pH 8.0, 150 mM NaCI, 0.1% NP40, 0.5 mM EDTA, 1 mM DTT).

The target protein was cleaved and released from the beads as follows. The washed Econocolumn was filled with 1 ml TEV cleavage buffer and 30 µl TEV protease and rotated in a 16°C incubator for 2 hours. The eluate was recovered by gravity flow.

The second affinity step (Calmodulin affinity step) was performed as follows:
The previous eluate was mixed with 3 ml of IPP 150-Calmodulin binding buffer (10 mM β-mercaptoethanol, 10 mM Tris-CI pH 8.0, 150 mM NaCl, 1 mM Mg-acetate, 1 mM imidazole, 2 mM CaCl₂, 0.1% NP40). The appropriate amount of CaCl2 was further added to block the EDTA coming from the TEV cleavage buffer. This mix was rotated for 1 hour in an Econocolumn containing 200 pi of Calmodulin beads slurry (Stratagene 214303) previously washed with 5 ml IPP 150-Calmodulin binding buffer.

After washing with 30 ml of IPP 150-Calmodulin binding buffer, protein complexes were eluted with 5 successive additions of 200 µl of IPP 150-Calmodulin elution buffer (10 mM β-mercaptoethanol, 10 mM Tris-CI pH 8.0, 150 mM NaCI, 1 mM Mg-acetate, 1 mM imidazole, 2 mM EGTA, 0.1 % NP40).

Samples were frozen in dry ice and stored at -80°C. Proteins were concentrated by TCA precipitation (A. Bensadoun and D. Weinstein (1976), Anal. Biochem. 70, 241). The proteins were detected by polyacrylamide gel electrophoresis with subsequent staining of the gel with Coomassie blue. The result of the protein purification, a gel of which is depicted in Fig.1 demonstrates that the strategy employed is highly efficient. All the expected protein subunits which number 24 in this case can be detected.

### Example 2

The same procedure was used for two other protein or protein-RNA complexes from yeast where all expected protein subunits were detected using the method of the invention. Those are the CBC (Cap Binding Complex) and the U1 snRNP. The purified U1 snRNP is depicted in Figures 2 and 3. The CBC complex has been shown to be still active and the purity was good in all cases. This method is relatively cheap and not very time-consuming, since it can be done in one day. Concerning the U1 snRNP, it is noteworthy that Neubauer et al. (Proc. Natl. Acad. Sci. USA 1997, 94, 385-390) carried out a purification of the same complex (U1 snRNP) extracted from 16 L of culture. The proteins of the complex of interest were then only visible by silver staining and several contaminants were still observed.

## Claims

1. Method for detecting and/or purifying biomolecule and/or protein complexes comprising the steps:
(a) providing an expression environment containing one or more heterologous nucleic acids encoding one or more subunits of a biomolecule complex, the subunits being fused to at least two different affinity tags, one of which consists of one or more IgG binding domains of Staphylococcus protein A,
(b) maintaining the expression environment under conditions that facilitate expression of the one or more subunits in a native form as fusion proteins with the affinity tags, and under conditions that allow the formation of a complex between the one or more subunits and possibly other components capable of complexing with the one or more subunits,
(c) detecting and/or purifying the one or more subunits by a combination of at least two different affinity purification steps each comprising binding the one or more subunits via one affinity tag to a support material capable of selectively binding one of the affinity tags and separating the one or more subunits from the support material after substances not bound to the support material have been removed.

2. Method for detecting and/or purifying biomolecule and/or protein complexes, comprising the steps:
(a) providing an expression environment containing one or more heterologous nucleic acids encoding at least two subunits of a biomolecule complex, each being fused to at least one of different affinity tags, one of which consists of one or more IgG binding domains of Staphylococcus protein A,
(b) maintaining the expression environment under conditions that facilitate expression of the two or more subunits in a native form as fusion proteins with the affinity tags, and under conditions that allow the formation of a complex between the two or more subunits and possibly other components capable of complexing with the two or more subunits,
(c) detecting and/or purifying the complex by a combination of at least two different affinity purification steps each comprising binding the two or more subunits via one affinity tag to a support material capable of selectively binding one of the affinity tags and separating the complex from the support material after substances not bound to the support material have been removed.

3. Method according to claim 1 or 2, wherein between the one or more subunits and one or more of the affinity tags a specific proteolytic cleavage site is present in one or more of the fusion proteins which facilitates the removal of one or more of the affinity tags.

4. Method according to claim 3, wherein the specific proteolytic cleavage site is an enzymatic cleavage site.

5. Method according to claim 4, wherein the specific proteolytic cleavage site is the cleavage site for TEV protease NIA.

6. Method according to claim 3, 4 or 5, wherein the proteolytic cleavage site is used to cleave the one or more of the subunits in step (c) from the IgG binding domain of Staphylococcus protein A bound to the support material.

7. Method according to claim 6, wherein the affinity purification of step (c) comprises:
(i) binding the one or more subunits via the one or more IgG binding domains of Staphylococcus to a support material capable of specifically binding the latter, removing substances not bound to the support material and separating the one or more subunits from the support material by cleaving off the IgG binding domains via the specific proteolytic cleavage site, and
(ii) binding the subunit via another affinity tag to a second support material capable of specifically binding the latter, removing substances not bound to the support material and separating the subunit from the support material.

8. Method according to claim 7, wherein step (ii) is carried out before step (i).

9. Method according to one of the previous claims, wherein one or more of the fusion proteins contain a second specific proteolytic cleavage site for the removal of one or more of the other affinity tags.

10. Method according to one of the previous claims, wherein one of the affinity tags consists of at least one calmodulin binding peptide.

11. Method according to claim 10, wherein a chemical agent is used to separate the one or more subunits from the support material.

12. Use of the method according to one of claims 1 to 11 for the detection and/or purification of substances capable of complexing with one or more of the fusion proteins.

## Patentansprüche

1. Verfahren zum Nachweisen und/oder Reinigen von Biomolekül- und/oder Proteinkomplexen, umfassend die Schritte:
(a) Bereitstellen einer Expressionsumgebung, die eine oder mehrere heterologe Nukleinsäuren enthält, die für eine oder mehrere Untereinheiten eines Biomolekülkomplexes codieren, wobei die Untereinheiten mit wenigstens zwei verschiedenen Affinitätstags fusioniert sind, von denen einer aus einer oder mehreren IgG-Bindungsdomänen des Staphylococcus-Proteins A besteht,
(b) Aufrechterhalten der Expressionsumgebung unter Bedingungen, welche die Expression der einen oder mehreren Untereinheiten als Fusionsproteine mit den Affinitätstags in einer nativen Form erleichtern, und unter Bedingungen, welche die Bildung eines Komplexes zwischen den einen oder mehreren Untereinheiten und möglicherweise anderen Komponenten, die einen Komplex mit der einen oder mehreren Untereinheiten ausbilden können, ermöglichen,
(c) Nachweisen und/oder Reinigen der einen oder mehreren Untereinheiten durch eine Kombination von wenigstens zwei verschiedenen Affinitätsreinigungsschritten, wobei jeder das Binden der einen oder mehreren Untereinheiten über einen Affinitätstag an ein Trägermaterial, das fähig ist, einen der Affinitätstags selektiv zu binden und das Abtrennen der einen oder mehreren Untereinheiten vom Trägermaterial, nachdem nicht an das Trägermaterial gebundene Substanzen entfernt wurden, umfasst.

2. Verfahren zum Nachweisen und/oder Reinigen von Biomolekül- und/oder Proteinkomplexen, umfassend die Schritte:
(a) Bereitstellen einer Expressionsumgebung, die eine oder mehrere heterologe Nukleinsäuren enthält, die für wenigstens zwei Untereinheiten eines Biomolekülkomplexes codieren, wobei jede mit wenigstens einem unterschiedlichen Affinitätstag fusioniert ist, von denen einer aus einer oder mehreren IgG-Bindungsdomänen des Staphylococcus-Proteins A besteht,
(b) Aufrechterhalten der Expressionsumgebung unter Bedingungen, welche die Expression der zwei oder mehreren Untereinheiten als Fusionsproteine mit den Affinitätstags in einer nativen Form erleichtern und unter Bedingungen, welche die Bildung eines Komplexes zwischen den zwei oder mehreren Untereinheiten und möglicherweise anderen Komponenten, die einen Komplex mit den zwei oder mehreren Untereinheiten ausbilden können, ermöglichen,
(c) Nachweisen und/oder Reinigen des Komplexes durch eine Kombination von wenigstens zwei verschiedenen Affinitätsreinigungsschritten, wobei jeder das Binden der zwei oder mehreren Untereinheiten über einen Affinitätstag an ein Trägermaterial, das fähig ist, einen der Affinitätstags selektiv zu binden und das Abtrennen des Komplexes von dem Trägermaterial, nachdem nicht an das Trägermaterial gebundene Substanzen entfernt wurden, umfasst.

3. Verfahren nach Anspruch 1 oder 2, wobei in einem oder mehreren der Fusionsproteine eine spezifische proteolytische Spaltstelle zwischen den einen oder mehreren Untereinheiten und einem oder mehreren der Affinitätstags vorliegt, welche die Entfernung von einem oder mehreren der Affinitätstags erleichtert.

4. Verfahren nach Anspruch 3, wobei die spezifische proteolytische Spaltstelle eine enzymatische Spaltstelle ist.

5. Verfahren nach Anspruch 4, wobei die spezifische proteolytische Spaltstelle die Spaltstelle für die TEV-Protease NIA ist.

6. Verfahren nach Anspruch 3, 4 oder 5, wobei die proteolytische Spaltstelle verwendet wird, um die eine oder mehreren Untereinheiten in Schritt (c) von der an das Trägermaterial gebundenen IgG-Bindungsdomäne des Staphylococcus-Proteins A abzuspalten.

7. Verfahren nach Anspruch 6, wobei die Affinitätsreinigung von Schritt (c) umfasst:
(i) Binden der einen oder mehreren Untereinheiten über die eine oder mehreren IgG-Bindungsdomänen von Staphylococcus an ein Trägermaterial, das fähig ist, letztere spezifisch zu binden, Entfernen von nicht an das Trägermaterial gebundenen Substanzen und Abtrennen der einen oder mehreren Untereinheiten von dem Trägermaterial durch Abspalten der IgG-Bindungsdomänen über die spezifische proteolytische Spaltstelle, und
(ii) Binden der Untereinheit über einen anderen Affinitätstag an ein zweites Trägermaterial, das fähig ist, letzteren spezifisch zu binden, Entfernen von nicht an das Trägermaterial gebundenen Substanzen und Abtrennen der Untereinheit vom Trägermaterial.

8. Verfahren nach Anspruch 7, wobei der Schritt (ii) vor dem Schritt (i) durchgeführt wird.

9. Verfahren nach einem der vorhergehenden Ansprüche, wobei ein oder mehrere der Fusionsproteine eine zweite spezifische proteolytische Spaltstelle zur Entfernung von einem oder mehreren der anderen Affinitätstags aufweist.

10. Verfahren nach einem der vorhergehenden Ansprüche, wobei einer der Affinitätstags aus wenigstens einem Calmodulin-Bindungspeptid besteht.

11. Verfahren nach Anspruch 10, wobei ein chemisches Agens zur Abtrennung der einen oder mehreren Untereinheiten von dem Trägermaterial verwendet wird.

12. Verwendung des Verfahrens nach einem der Ansprüche 1 bis 11 zum Nachweis und/oder zur Reinigung von Substanzen, die einen Komplex mit einem oder mehreren der Fusionsproteine bilden können.

## Revendications

1. Procédé de détection et/ou de purification de complexes de biomolécules et/ou de protéines comprenant les étapes consistant à :
(a) fournir un environnement d'expression contenant un ou plusieurs acides nucléiques hétérologues codant pour une ou plusieurs sous-unités d'un complexe de biomolécules, les sous-unités étant fusionnées à au moins deux marqueurs d'affinité différents, dont l'un est constitué d'un ou plusieurs domaines de liaison aux IgG de la protéine A de Staphylococcus,
(b) maintenir l'environnement d'expression dans des conditions facilitant l'expression de la ou des sous-unités sous forme native en tant que protéines de fusion avec les marqueurs d'affinité, et dans des conditions permettant la formation d'un complexe entre la ou les sous-unités et éventuellement d'autres composants capables de former des complexes avec la ou les sous-unités,
(c) détecter et/ou purifier la ou les sous-unités par une combinaison d'au moins deux étapes différentes de purification par affinité, comprenant chacune la liaison de la ou des sous-unités par l'intermédiaire d'un marqueur d'affinité à un matériau support capable de lier sélectivement l'un des marqueurs d'affinité et la séparation de la ou des sous-unités du matériau support après l'élimination des substances non liées au matériau support.

2. Procédé de détection et/ou de purification de complexes de biomolécules et/ou de protéines comprenant les étapes consistant à :
(a) fournir un environnement d'expression contenant un ou plusieurs acides nucléiques hétérologues codant pour au moins deux sous-unités d'un complexe de biomolécules, fusionnées chacune à au moins un marqueur parmi différents marqueurs d'affinité, dont l'un est constitué d'un ou plusieurs domaines de liaison aux IgG de la protéine A de Staphylococcus,
(b) maintenir l'environnement d'expression dans des conditions facilitant l'expression des deux sous-unités ou plus sous forme native en tant que protéines de fusion avec les marqueurs d'affinité, et dans des conditions permettant la formation d'un complexe entre les deux sous-unités ou plus et éventuellement d'autres composants capables de former des complexes avec les sous-unités ou plus,
(c) détecter et/ou purifier le complexe par une combinaison d'au moins deux étapes différentes de purification par affinité, comprenant chacune la liaison des deux sous-unités ou plus par l'intermédiaire d'un marqueur d'affinité à un matériau support capable de lier sélectivement l'un des marqueurs d'affinité et la séparation du complexe du matériau support après l'élimination des substances non liées au matériau support

3. Procédé selon la revendication 1 ou 2, dans lequel entre la ou les sous-unités et le ou les marqueurs d'affinité, un site de clivage protéolytique spécifique est présent dans une ou plusieurs des protéines de fusion, lequel facilite l'élimination d'un ou plusieurs des marqueurs d'affinité.

4. Procédé selon la revendication 3, dans lequel le site de clivage protéolytique spécifique est un site de clivage enzymatique.

5. Procédé selon la revendication 4, dans lequel le site de clivage protéolytique spécifique est le site de clivage de la protéase NIA du TEV.

6. Procédé selon la revendication 3, 4 ou 5, dans lequel le site de clivage protéolytique est utilisé pour cliver la ou les sous-unités à l'étape (c) du domaine de liaison aux IgG de la protéine A de Staphylococcus lié au matériau support.

7. Procédé selon la revendication 6, dans lequel l'étape de purification par affinité (c) comprend les étapes consistant à :
(i) lier la ou les sous-unités par l'intermédiaire du ou des domaines de liaison aux IgG de Staphylococcus à un matériau support capable de lier spécifiquement ces derniers, éliminer les substances non liées au matériau support et séparer la ou les sous-unités du matériau support en clivant les domaines de liaison aux IgG par l'intermédiaire du site de clivage protéolytique spécifique, et
(ii) lier la sous-unité par l'intermédiaire d'un autre marqueur d'affinité à un second matériau support capable de lier spécifiquement ce dernier, éliminer les substances non liées au matériau support et séparer la sous-unité du matériau support.

8. Procédé selon la revendication 7, dans lequel l'étape (ii) est effectuée avant l'étape (i).

9. Procédé selon l'une des revendications précédentes, dans lequel une ou plusieurs des protéines de fusion contiennent un second site de clivage protéolytique spécifique pour l'élimination d'un ou plusieurs des autres marqueurs d'affinité.

10. Procédé selon l'une des revendications précédentes, dans lequel l'un des marqueurs d'affinité est constitué d'au moins un peptide de liaison de la calmoduline.

11. Procédé selon la revendication 10, dans lequel un agent chimique est utilisé pour séparer la ou les sous-unités du matériau support.

12. Utilisation du procédé selon l'une des revendications 1 à 11 pour la détection et/ou la purification de substances capables de former des complexes avec une ou plusieurs des protéines de fusion.
